# EUROPEAN PATENT SPECIFICATION

(11) **EP 4 543 296 B1**
(45) Date of publication and mention of the grant of the patent: **29.07.2026**
(21) Application number: 22744578.0
(22) Date of filing: 22.06.2022
(51) Int. Cl.: A61B 5/15, A61M 39/00, A61B 5/0215, A61B 5/021, A61M 25/00, A61M 1/00, A61B 5/00

(54) **SYSTEM FOR DETERMING A CLOTTING EVENT IN AN ARTERIAL LINE**
SYSTEM ZUR BESTIMMUNG EINES GERINNUNGSEREIGNISSES IN EINER ARTERIENLEITUNG
SYSTÈME DE DÉTERMINATION D'ÉVÉNEMENT DE COAGULATION DANS UN CATHÉTER ARTÉRIEL

(43) Date of publication of application: 30.04.2025
(73) Proprietor: Becton, Dickinson and Company, Franklin Lakes, NJ 07417-1880 (US)
(72) Inventor: VAN GOUDOEVER, Jeroen, Franklin Lakes, NJ 07417 (US); KUIJKENS, Hans Jean Paul, Franklin Lakes, NJ 07417 (US); REUDERINK, Boris, Franklin Lakes, NJ 07417 (US)
(74) Representative: Venner, Julia Ann
(86) International application number: PCT/US2022/034485
(87) International publication number: WO 2023/249621

(56) References cited:
- US-A1- 2017 258 975
- US-A1- 2021 322 026
- US-B1- 6 623 443

## Description

### TECHNICAL FIELD

The present disclosure relates to a method for notifying a clotting event in an arterial line of a human being, a corresponding system and corresponding computer program.

### BACKGROUND OF THE INVENTION

An arterial line is a small indwelling catheter that is inserted into an artery of a human being, such as the radial artery. Arterial lines are commonly used in critical care. They allow to repeatedly draw blood easily without having to stick the patient with a needle again and again, and allow to continuously assess blood pressure, to for instance, continuously display waveform and/or pressure from within the artery, for instance on a bedside monitor. Further, other hemodynamic parameters such as stroke volume, cardiac output, Systemic Vascular Resistance (SVR), maximum pressure gradient (max. dp/dt) and derived parameters such as Pulse Pressure Variation (PPV), Stroke Volume Variation (SVV), or the Hypotension Prediction Index (HPI) developed by Edwards Lifesciences (Irvine, CA), are available.

During a measurement using an arterial line, clotting in the arterial line can occur, even though a continuous flush system is used aimed to prevent clotting. The quality of the arterial line and thus of the measurement deteriorates. However, the recognition of the onset or presence of a clotting event is difficult. Usually blood pressure readings, for instance systolic or diastolic pressure readings or a mean arterial pressure, stay close to the original value well within the normal physiological range even when a clotting event occurs.

The waveform cannot be easily assessed by the anesthesiologist due to the size of the blood pressure waveform signal, for instance on the patient monitor screen. Because of the gradual reduction in pulse pressure, a slow trend is often visible in, for example Stroke Volume (SV). However, the reduction in SV could also have another physiological origin, so is often not recognized (timely) by the anesthesiologist. In applications such as peri- operative fluid management, such a slow degradation of measured signals can even be highly misleading and could cause wrong diagnosis and unwanted interventions.

US 2017/258975 describes a system for detecting an occlusion in the tubing of a dialysis system. An occlusion alarm is triggered based on correlation or comparison of measured pressure values with predetermined pressure values.

US 2021/0322026 describes systems and methods for blood flow control. In some variations, a blood flow control system may comprise a blood flow control device which may be placed within a body of a patient and may comprise an expandable member and a sensor. The sensor may be configured to measure at least one of a physiologic condition of the patient and a pressure associated with the expandable member. The blood flow control system may include at least one controller communicably coupled to the sensor to: receive data indicative of at least one of the physiologic condition of the patient and the pressure associated with the expandable member from the sensor, compare the received data with target data, identify at least one error based on the comparison, and in response to identifying the error, inhibit at least one function of the blood flow control system.

### SUMMARY

It has therefore been an object of the present invention, to increase the patient safety during interventions, in which an arterial line is inserted into the patient.

The presently claimed invention is set out in the independent claim. Particular embodiments of this aspect are set out in the dependent claims. Any subject matter contained herein that does not fall within the scope of the appended claims is considered as being useful for understanding the invention.

In an aspect of the disclosure useful for understanding the presently claimed invention, a method for notifying a clotting event in an arterial line of a human being is suggested. The method comprises a step of providing a hemodynamic signal indicative of an arterial blood pressure waveform in the arterial line.

The hemodynamic signal can be a digital or analogue representation of the arterial blood pressure waveform itself. In other examples, the hemodynamic signal can be filtered or processed, for instance averaged over a time which is significantly shorter than a duration of a heartbeat, before being provided.

In some embodiments, the hemodynamic signal is provided substantially in real time during the intervention, i.e., during a time in which the arterial line is inserted into the patient. In this case, the hemodynamic signal is preferentially provided continuously over the entire intervention period or parts thereof. In other examples, the hemodynamic signal can be previously recorded and therefore be indicative of the arterial blood pressure waveform of a previous intervention. The hemodynamic signal can in such examples be provided by means of wired or wireless data transmission, originating from a storage means such as a server, for instance.

The hemodynamic signal being provided does in the context of the present invention not imply any spatial relationship between the origin of the hemodynamic signal and the destination it is provided to. In some examples, the hemodynamic signal can be provided to a processing device right next to the patient. In other examples, the hemodynamic signal can be provided to a processing device, such as a server, which is remote from the patient. Any feasible form of providing the hemodynamic signal is thus contemplated in the context of the present disclosure.

The method further comprises a step of determining a hemodynamic contraction parameter related to a contraction of the heart based on the hemodynamic signal.

The hemodynamic contraction parameter includes one or a combination of a plurality of individual hemodynamic parameters. A parameter is related to the contraction of the heart when its corresponding features originate from the contraction of the left ventricle, which creates a pulse wave. The pulse wave causes a sudden rise in arterial pressure and a sudden rise in forward flow through the artery.

The method further comprises a step of determining a hemodynamic relaxation parameter related to a relaxation of the heart based on the hemodynamic signal.

During the relaxation period of the heart, a diastolic runoff in arterial blood pressure occurs until the next heartbeat starts a new pulsation. The hemodynamic relaxation parameter includes one or a combination of a plurality of hemodynamic parameters, which can be derived from the relaxation of the heart.

Both the hemodynamic contraction parameter and the hemodynamic relaxation parameter can be predefined, for instance, be static for the operation of the method. In other examples, the hemodynamic contraction parameter and/or the hemodynamic relaxation parameter can change over time, as will be detailed below.

Therefore, in some embodiments, the steps or mathematical rules for obtaining the hemodynamic contraction parameter and/or the hemodynamic relaxation parameter can be provided in order to determine the corresponding parameter. All feasible forms known to the skilled person to implement the determination of a hemodynamic parameter are contemplated.

The method further comprises a step of determining a clotting event in the arterial line in case the hemodynamic contraction parameter indicates a decrease of contractility, and the hemodynamic relaxation parameter indicates an increase of contractility.

It is a key finding of the inventors of the present disclosure that an apparent contradiction in hemodynamic parameters distal of a clotted artery can be indicative of the occurrence of a clotting event in the arterial line. Since the hemodynamic contraction parameter indicates a decrease of contractility and the hemodynamic relaxation parameter indicates an increase of contractility, both parameters contradict each other. This situation is recognized according to the present disclosure as the occurrence of a clotting event.

The method further comprises a step of providing a signal notifying of the determined clotting event.

In case a clotting event is detected, the present method foresees a step of notifying of the determined clotting event. Any feasible kind of notification is envisaged by the present disclosure. In one example, the signal notifying of the determined clotting event results in a message or other visually recognizable indication on the patient monitor, which is generally known to illustrate the pulse pressure wave during, for instance, a critical care intervention.

In other examples, the signal is notified to a server or another entity and used to generate a visual, audible or other form of noticeable notification to the medical care giver.

The signal notifying of the determined clotting event can thus facilitate a timely identification of a clotting event and result in - automatic or manual - flushing of the arterial line in order to resolve the detected clotting.

While in this embodiment the step of providing a signal notifying of the determined clotting event is described as an integral part of the method, in other embodiments a method which does not employ the step of notifying is also beneficial. For instance, the result of the determination of the clotting event can then be further processed.

In a preferred embodiment, the hemodynamic contraction parameter includes at least one hemodynamic parameter derivable from the arterial blood pressure waveform between a begin of the systolic phase and a closure of the aortic valve at the end of systole, wherein the hemodynamic contraction parameter in particular includes at least one of
- a maximum change in blood pressure during systole,
- an end systolic pressure,
- an area under the arterial blood pressure waveform curve above end diastolic pressure between the begin of the systolic phase and the closure of the aortic valve at the end of systole.

The hemodynamic contraction parameter thus is related to the heart's contraction, because a pulse wave is created when the left ventricle is contracting. This pulse wave causes a sudden rise in arterial pressure and a sudden rise in forward flow through the artery. Any parameter suitable of characterizing the build-up of pressure in the artery during systole can thus be part of the hemodynamic contraction parameter according to this embodiment. Further feasible parameters include first, second etc. derivatives, a maximum derivative and so on of the parameters listed above. It should be noted that the above-mentioned list of parameters is not complete and the person skilled in the art appreciates that additional parameters can be employed, either alone or in combination, in order to obtain the hemodynamic contraction parameter.

In a preferred embodiment, the hemodynamic relaxation parameter includes at least one of an end-diastolic pressure; a mean-arterial pressure; and an interbeat interval.

The hemodynamic relaxation parameter is thus a parameter related to the relaxation period of the arterial blood pressure waveform, during which the arterial blood pressure decreases until a next heartbeat starts a new pulsation. Expressed differently, all parameters which are not attributable to the contraction of the left ventricle may be regarded as parameters related to the relaxation period, this includes the interbeat interval. Also with regard to the hemodynamic relaxation parameter it should be emphasized that further additional parameters are well-known to the person skilled in the art and all these parameters can either alone or in combination with further hemodynamic relaxation parameters be used as the hemodynamic relaxation parameter in this embodiment.

In a preferred embodiment, at least one of the hemodynamic contraction parameter and the hemodynamic relaxation parameter is determined as an average over a predefined period of time, in particular as a 20 second average.

Determining the hemodynamic contraction parameter and/or the hemodynamic relaxation parameter as an average over a predetermined period of time allows to have a more robust value of the corresponding parameter. In other words, outlying extreme values or measurement errors will not lead to the determination of a clotting event unintentionally. The predefined period of time can be a static amount of time such as the example of, for instance, 20 seconds. In other examples, the period of time may be defined as the number of subsequent heartbeats, for instance, the average may always be taken over a certain number of heartbeats such as 15 or 20 heartbeats.

In a preferred embodiment, the determining as an average includes at least one of a filtering process and determining a moving average.

Filtering processes and moving averages are well-known and allow simple to implement ways of determining average values. In other examples, other forms of determining averages are also contemplated.

In a preferred embodiment, a decrease or increase of the hemodynamic contraction parameter and the hemodynamic relaxation parameter is indicated based on a comparison of two subsequent values of the hemodynamic contraction parameter and the hemodynamic relaxation parameter, respectively.

In this embodiment, the determination of subsequent values of the hemodynamic contraction parameter and the hemodynamic relaxation parameter, respectively, allows a simple comparison of a trend or change in the respective parameter. Subsequent values can be directly neighbouring values of the hemodynamic contraction parameter and the hemodynamic relaxation parameter, respectively, or intermediate values between the two subsequent values can exist, i.e., a gap exists between the first and second respective parameter.

In particular, it is preferred that the underlying period of time for the subsequent values of the hemodynamic contraction parameter and the hemodynamic relaxation parameter is sufficiently apart such that sufficient change in the respective parameter can occur. Thereby, slight trends or changes which are induced in the hemodynamic contraction parameter and the hemodynamic relaxation parameter, which would not result in a detected clotting for directly neighbouring heartbeats, can be detected if the change over a longer period of time is sufficiently large. For example, even though the moving average would be refreshed with every heartbeat, e.g., approximately every second, it would be preferred to compare the moving average which is more than one heartbeat apart, for instance, two subsequent values, for which the underlying time period would be 20 seconds apart.

In a preferred embodiment, the method further comprises, after determining a clotting event, the steps of
- recognizing or recommending a flushing event of the arterial line, and
- verifying a clotting event in case a change in at least one predetermined hemodynamic parameter, in particular of maximum change in blood pressure during systole or end systolic pressure, before and after the flushing event exceeds a predetermined flushing threshold.

The step of recognizing or recommending a flushing event of the arterial line can be performed substantially simultaneously with the step of providing a signal notifying of the determined clotting event. For example, the recommendation of the flushing event can result in an additional message or notification on the patient monitor indicating to the anesthesiologist to carry out a flushing of the arterial line. In other examples, the recommendation of a flushing event can include the generation of a flushing signal, which is then used to initiate an automated flushing of the arterial line. Flushing of the arterial line will increase the reliability of the measurement results relying on the pulse pressure wave as measured through the arterial line.

In a preferred embodiment, the flushing event of the arterial line is determined based on a significant change in at least one of maximum pressure gradient and systolic pressure.

A flushing event can be seen as a sharp peak in pressure to, for example, 300 mmHg or higher due to a pressure bag providing automated infusion to prevent clotting. The higher the location of the pressure bag, the sharper the recognizable peak in pressure will be.

If after such a flushing event, systolic pressure, diastolic pressure and other hemodynamic parameters are suddenly restored to values similar to values before the event and different from values during the event, the clotting can be determined as a true positive event.

Scoring or proving the performance of the method according to the present disclosure is possible based on an offline analysis on clinical data with arterial blood pressure waveforms. The determination of a clotting event and the subsequent provided signal notifying of the determined clotting event can be tested and verified when combining with the evidence that the arterial line was indeed subsequently flushed. If, however, the signal notifying the determined clotting event is provided, i.e., a clotting event is determined, but no flushing event was detected within a reasonable time period after the determination of the clotting event in the offline analysis on clinical data, the trigger is preferentially classified as a false positive determination.

A reasonable time period in this context may be, for instance, 5 minutes. It is therefore possible to prove the performance of a clotting detection algorithm. Since there is a group of clotting events which are not recognized until now by anesthesiologists as clotting events in practise, the true positive events may, for instance, be weighted higher than the false positive events for the sake of scoring the algorithm performance. Obviously, the number of false positives should not be too high, because of potential alarm fatigue of, for instance, the anesthesiologist.

Additionally, in a preferred embodiment, an oscillation in the system after recognizing a flushing event can be used to determine a damping, wherein the damping is characterized by a frequency instability which occurs. The determined damping can then advantageously be used to characterize the system.

In a preferred embodiment, the method further comprises selecting, out of a list of available hemodynamic parameters, a subset of the available hemodynamic parameters as individual clotting parameters, wherein a hemodynamic parameter classifies as individual clotting parameter in case a change between "before" and "after" the flushing event exceeds a predefined threshold.

The selection of generally suitable hemodynamic parameters as the hemodynamic contraction parameter and the hemodynamic relaxation parameter, respectively, can, as mentioned above, be based on a scoring of the performance of the respective parameters based on an offline analysis on clinical data.

However, clotting events tend to occur a plurality of times in monitored patients during, for instance, intensive care observation. For such patients, the individual parameters for determining the clotting events may be different from the generic parameters determined on, for instance, the offline analysis on clinical data. It is therefore preferred that after the occurrence of a clotting event the suitability of alternative or additional parameters is evaluated for the sake of improving the prediction and determination of future clotting events for the same patient. Accordingly, in this embodiment, individual adaptation or prediction for a clotting event based on actual data is performed, such that subsequent clotting events can be determined more reliably or even earlier.

In a preferred embodiment of the method for determining a subsequent clotting event, at least one of the hemodynamic contraction parameter and the hemodynamic relaxation parameter includes a hemodynamic parameter included in the individual clotting parameters.

In this embodiment, the individual clotting parameter selected from the set of individual clotting parameters can be used additionally, e.g., in the form of a parameter combination, or alternatively to the previously considered hemodynamic contraction parameter or hemodynamic relaxation parameter, respectively. In some embodiments, the individual clotting parameters are ranked, wherein a highest ranked parameter shows a sign of the clotting event as early and reliably as possible. The ranking may be conducted, for example, using well-known statistics methods on the hemodynamic signal.

In a preferred embodiment, at least one of the hemodynamic contraction parameter and the hemodynamic relaxation parameter is adapted using a machine learning prediction algorithm.

The disclosure is not limited to a particular machine learning prediction algorithm and all machine learning prediction algorithms available to the skilled person can be employed. The machine learning prediction algorithm can be employed for both determining a generic hemodynamic contraction parameter and/or hemodynamic relaxation parameter, which is generically used as an initial parameter for the method according to the present disclosure. In other examples, the machine learning prediction algorithm can also be used to adapt the respective parameters during monitoring of the patient.

In a preferred embodiment, the hemodynamic contraction parameter indicates a decrease of contractility in case a change in the hemodynamic contraction parameter exceeds a decrease threshold and the hemodynamic relaxation parameter indicates an increase of contractility in case a change in the hemodynamic relaxation parameter exceeds an increase threshold. At least one of the increase threshold and the decrease threshold is adjusted based on previously determined clotting events, in particular based on past verified clotting events.

According to this embodiment, the thresholds for recognizing the increase and decrease of contractility, respectively, can be adjusted or learned from the fact that there was a flush and based on whether or not that flush caused significant changes in the hemodynamic parameters. Thereby, the sensitivity of the method according to the present disclosure can be improved.

In a preferred embodiment, the method further comprises the step of adjusting at least one of the increase threshold and the decrease threshold using the hemodynamic contraction parameter and the hemodynamic relaxation parameter before and after flushing the arterial line, respectively.

In a preferred embodiment, prediction parameters including the increase threshold and the decrease threshold are adapted using a machine learning prediction algorithm.

Likewise to the use of the machine learning prediction algorithm, which was described above with regard to the definition or combination of the hemodynamic contraction parameter and the hemodynamic relaxation parameter, the machine learning prediction algorithm can be employed in adjusting the increase threshold and the decrease threshold.

In a preferred embodiment, the method further comprises, a step of determining a frequency content of the provided hemodynamic signal, wherein in the step of determining the clotting event, the clotting event is determined additionally based on the determined frequency content, wherein in particular the clotting event is determined in case the frequency content or a change of the frequency content exceeds a predetermined threshold.

It is recognized that the frequency content of the hemodynamic signal can in addition to the hemodynamic contraction parameter and the hemodynamic relaxation parameter be evaluated to determine the occurrence of a clotting event in an arterial line. It showed to be of particular benefit and to increase the reliability of the proposed method to combine the analysis of the hemodynamic contraction/relaxation parameter with the analysis of the frequency content.

The frequency content is preferentially determined based on the hemodynamic signal, which is indicative of a blood pressure waveform, after transformation into frequency domain such as using fourier or other suitable transformations. The frequency content is preferentially determined as an integral over a specific frequency range or the complete frequency range.

A change in the frequency content is preferentially determined for two subsequent values of the frequency content, wherein the subsequent values preferentially include a time gap between them, as has been described above with regard to subsequent values of the hemodynamic contraction parameter and the hemodynamic relaxation parameter. Thereby, slow trends or shifts accumulate to a significant change which can be noticed and will not be left undetected. It should be noted that all averaging processing known to the skilled person and all further processing described above with regard to the hemodynamic contraction parameter and the hemodynamic relaxation parameter can likewise be applied to the frequency content.

In a preferred embodiment the frequency content is determined as a sum of the frequency contents within a frequency range of interest, the frequency range of interest including a frequency of interest and in particular a resonance such as a natural frequency of the arterial line, wherein the frequency content is particularly preferably determined as a relative value scaled with a sum of the frequency contents over all frequencies.

Preferentially, the frequency of interest is determined as the frequency having the largest amplitude. In some embodiments, the frequency of interest can be predetermined and fixed, in other embodiments, the frequency of interest is variable and may be adapted in accordance with the presently largest amplitude.

Since the frequency content is preferentially scaled relative to a total sum over all frequencies, the frequency content of the frequency range of interest can be compared over time, in particular a change of the frequency content over time can be analysed more accurately.

In a preferred embodiment the frequency range of interest is determined relative to the frequency of interest and in particular includes frequency within 50% to 200% of the frequency of interest and/or includes frequencies with amplitudes above 50% of the amplitude of the frequency of interest. It should be noted that also other ways of defining suitable ranges of interest are contemplated.

In a preferred embodiment the method further comprises, after determining a clotting event, the steps of executing a flush test, the flush test comprising an opening of a pressurised reservoir and a sudden stop of the flow resulting in a sudden change of blood pressure, evaluating oscillations of the arterial line system based on the provided hemodynamic signal indicative of the arterial blood pressure waveform for a period following the sudden stop, the oscillations in the hemodynamic signal being induced by the sudden change of blood pressure, and assessing damping and natural frequency of the arterial line system based on the evaluated oscillations.

In this embodiment, the evaluation of the flush test can be used to confirm the presence of a clotting event and, for instance, increase the reliability of the proposed method.

The period following the sudden stop can include, for instance, a time period of several seconds, for instance 3 seconds or 10 seconds, or a number of subsequent heart beats. Of course, other ways of determining a suitable duration of the time period are contemplated.

For this time period, for instance, a damping factor or also known as damping ratio can be derived based on the damping of the detectable oscillations. The damping factor describes how the oscillations decay over time. The damping factor can then, for instance, be compared to a predetermined clotting threshold and in case the damping factor exceeds the predetermined clotting threshold the presence of a clotting event can be confirmed. The damping can therefore be advantageously used to confirm the clotting of the arterial line and therefore allows building an even more robust system.

In addition, at least one dominant frequency can be determined for the induced oscillations, which is referred to as natural frequency. Preferentially, also the frequency value of the natural frequency can be evaluated to verify the presence of a clotting event. For instance, the natural frequency can be compared with a predetermined frequency and the presence of a clotting event can be verified in case the natural frequency deviates more than a predetermined threshold from the predetermined frequency. The predetermined threshold can be absolute or relative values, such as 10 Hz or 10%, of course without being limited.

In a preferred embodiment the flush test executed in the step of executing includes at least one of an automated fast flush, in which the opening of the pressurized reservoir and/or the sudden stop of the flow is automatically operated using an automated flushing means capable of inducing a flush which can be a time wise short injection of a liquid, preferably a saline solution, with pressure into the arterial line, a manual fast flush, in which an instruction to perform the manual opening of the pressurized reservoir and/or the sudden stop of the flow is signalled to an operator, and a manual tapping test, wherein the tapping test comprises increasing once or several times the blood pressure by applying an increased pressure through the arterial line, for instance by tapping on flexible tubing connected to the arterial line, preferentially with a sudden release of the applied pressure, wherein the manual tapping test includes signalling an instruction to perform the manual tapping test to the operator.

The fast flush test allows to determine in vivo the natural frequency and damping coefficient of an invasive blood pressure monitoring system like an arterial line system. For the fast flush test a pressurized reservoir, where the pressure can preferentially be generated by gravity or by mechanical pressure, is connected to the arterial line through an openable and closable connection. The pressurize reservoir is preferentially an infusion bag, where the pressure can be changed by changing the height at which the infusion bag is held. The pressure in the pressurized reservoir is preferably approximately 200mm Hg or more. This pressurized reservoir can be opened for a short time, preferentially about 3 seconds or less such as only 1 second, in order to increase the blood pressure in the arterial system. By suddenly closing the pressurised reservoir, the blood pressure abruptly decreases and induces oscillations in the hemodynamic signal for up to a few seconds after the pressurized reservoir was closed.

Of course the pressure values and time values are just examples and other values of these parameters, for instance dynamically determined values of these parameters, can be employed by the skilled person.

Tapping the arterial line with for instance a finger can also be used to induce oscillations in the hemodynamic signal, which can be used to assess damping and natural frequency of the arterial line system. By tapping the arterial line on a soft connection/tube, the volume of the arterial system gets reduced which results in an increased blood pressure. By quickly releasing the pressure on the connection/tube a sudden drop in blood pressure is achieved and oscillations in the hemodynamic signal are induced. These oscillations are present from the moment of the sudden decrease of the blood pressure and last for up to a few seconds.

The signalling of an instruction to perform the manual tapping test or the manual fast flush test to the operator can include any suitable form of visual, audible or otherwise perceptible signal to the operator. In this case, the method preferentially includes a step of determining that the signalled test was indeed conducted by the operator. Some examples include a spoken confirmation or an input into an interface, such as a touchscreen, which is provided by the operator that the test was indeed conducted. In other examples, the method automatically determines that the test was conducted based on the determined sudden rise in the pressure.

In a preferred embodiment the method further comprises, after determining a clotting event, the step of verifying that the determined clotting event is indeed a true clotting event based on the assessed damping and the natural frequency of the arterial system.

In a preferred embodiment the method in case of a verified true clotting event further comprises the step of executing an automated flushing wherein the arterial line comprises an automated flushing means capable of inducing a flush which can be a time wise short injection of a liquid, preferably a saline solution, with pressure into the arterial line in order to free the arterial line from clotted blood.

Flushing the arterial line with saline solution is estimated to free the arterial line from clotted blood.

The automated flushing preferentially operates the same pressurized container which can be used to carry out the fast flush. However, also other means suitable for flushing the arterial line as known in the art can be employed.

As an alternative to executing the automated flushing, for instance in case no automated flushing means is attached to the arterial line, an instruction to perform a manual flushing can be signalled to the operator.

In a preferred embodiment, the steps of the method are performed repeatedly, in particular continuously.

The presently claimed invention provides a system for notifying a clotting event in an arterial line of a human being . The system comprises
- a hemodynamic signal providing unit for providing a hemodynamic signal indicative of an arterial blood pressure waveform in the arterial line,
- a hemodynamic contraction parameter determination unit for determining a hemodynamic contraction parameter related to a contraction of the heart based on the hemodynamic signal,
- a hemodynamic relaxation parameter determination unit for determining a hemodynamic relaxation parameter related to a relaxation of the heart based on the hemodynamic signal,
- a clotting event determination unit for determining a clotting event in the arterial line in case the hemodynamic contraction parameter indicates a decrease of contractility and the hemodynamic relaxation parameter indicates an increase of contractility, and
- a notification unit for providing a signal notifying of the determined clotting event.

This system allows to achieve the same advantages and benefits as described above with regard to the methods for notifying a clotting event in an arterial line. Likewise, the system according to this aspect can be combined with any preferred embodiment of the described methods and thereby also achieve the same benefits.

The hemodynamic signal providing unit, the hemodynamic contraction parameter determination unit, the hemodynamic relaxation parameter determination unit, the clotting event determination unit, the frequency content determination unit and the notification unit can be implemented partly or completely as software and/or hardware. Suitable hardware includes general purpose processing units (CPU) or the like. The respective units may be physically provided at the same location or spatially distributed over different devices. For instance, one, more or all of the units can be implemented in a server. In other examples, one, more or all of the units can be integrated into the patient monitor, which is known to be used for monitoring the arterial blood pressure waveform of the arterial line. Further, suitable units configured to carry out the steps of the preferred embodiments of the methods according to the present disclosure can analogously be provided and implemented partly or completely as software and/or hardware.

Also disclosed herein is a computer program product comprising instructions is suggested which, when the program is executed by a computer, cause the computer to carry out the steps of the method of the first aspect of the present disclosure or a preferred embodiment thereof.

Also disclosed herein is a computer-readable data carrier carrying the computer program product of an aspect of the present disclosure is suggested. For instance the computer-readable data carrier is realized as a flash drive, e.g. a USB flash drive, a solid-state drive or on a hard disk drive, a cloud storage and the like.

The computer program product according to the present disclosure and the data carrier signal according to the present disclosure allow to achieve the same objectives as described in the context of the method according to the present disclosure. Even further, also the computer program product and the data carrier signal can advantageously be configured according to any of the described preferred embodiments of the method according to the present disclosure.

### BRIEF DESCRIPTION OF THE DRAWINGS

The disclosure and presently claimed invention will be explained by means of the non-limiting working examples depicted in the following figures. Specifically:
FIG. 1 schematically and exemplarily illustrates an arterial line setup.
FIG. 2 schematically and exemplarily illustrates an arterial blood pressure waveform.
FIG. 3 schematically and exemplarily illustrates a flow chart of a method according to the present disclosure.
FIG. 4 schematically and exemplarily illustrates a flow chart of a further method compatible with the present disclosure.
FIG. 5 schematically and exemplarily illustrates a system for notifying a clotting event.

### DETAILED DESCRIPTION

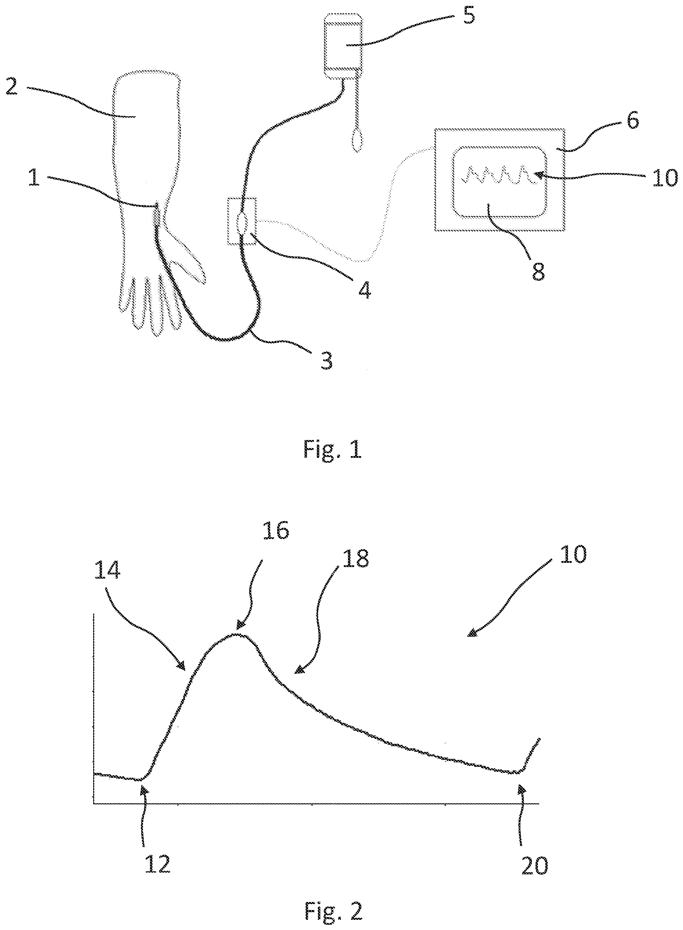

Fig. 1 schematically and exemplarily illustrates an intervention in which an arterial line 1 is inserted into an arm of a patient 2. In this example, the arterial line 1 is inserted into the radial arteria, while also a different arteria can be used in other examples. The arterial line 1 comprises a tubing 3 which is generally saline filled and non-compressible. The saline originates from a pressurized reservoir such as a pressure bag 5 which is generally located higher than the arterial line 1. In between pressure bag 5 and tubing 3 a pressure transducer 4 is located. The pressure transducer 4 is configured to provide a hemodynamic signal indicative of an arterial blood pressure waveform in the arterial line 1 to a patient monitor 6 in this example.

In some examples, the pressure transducer 4 includes an automatic flushing system for flushing the arterial line with saline from pressure bag 5. In other examples, the flushing system is manually activated.

In this example, patient monitor 6 comprises a display 8 on which a blood pressure waveform 10 is illustrated.

Fig. 2 schematically and exemplarily illustrates the arterial blood pressure waveform 10 for one single heartbeat in further detail. Fig. 2 illustrates a pressure on the vertical axis over time on the horizontal axis. It should be contemplated that also other forms of displaying the arterial blood pressure waveform are feasible.

Fig. 2 is only provided to illustrate the determination of the hemodynamic contraction parameter and the hemodynamic relaxation parameter from the hemodynamic signal according to the present disclosure. At a point 12, the aortic valve opens and the systolic phase begins. During a period 14, pressure increases as blood flows into the artery. At point 16, the maximum systolic pressure occurs. During a period 18, the heart valve closes and the pressure continuously falls as blood flows out of the artery. Eventually, at point 20, a minimum diastolic pressure can be obtained.

In some examples, the period between point 12 and point 16 is considered the systolic phase, parameters derived from this period can therefore be attributed to the contraction of the heart and referred to as hemodynamic contraction parameters. These parameters include, without being limited, the maximum change in blood pressure during period 14, the end systolic pressure at point 16 or an area under the arterial blood pressure waveform curve above end diastolic pressure at point 20 between the beginning of the systolic phase at point 12 and the closure of the aortic valve at the end of systole at point 18.

The parameters indicative of relaxation can include, without being limited, the end diastolic pressure at point 20, a mean arterial pressure as an average over the entire heartbeat and the interbeat interval.

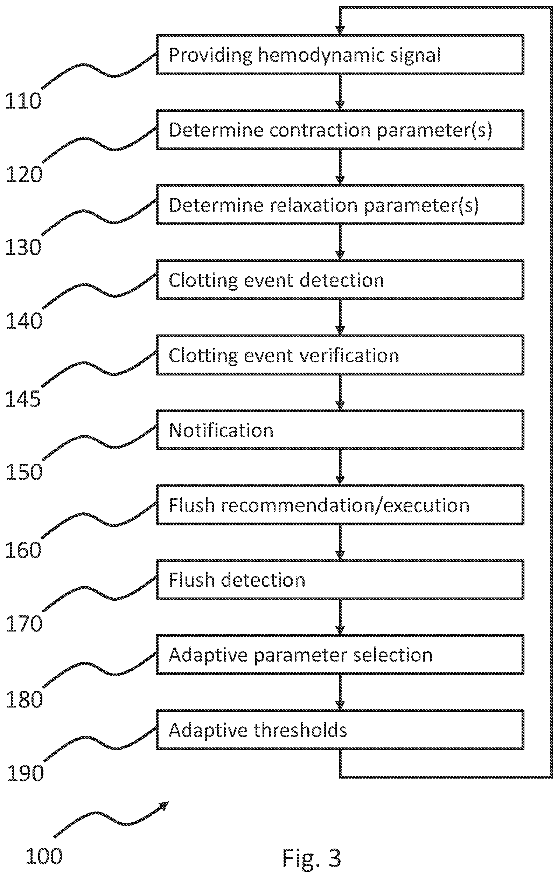

Fig. 3 schematically and exemplarily illustrates a flow chart of a method 100 for notifying a clotting event in an arterial line 1.

The method comprises a step 110 of providing a hemodynamic signal indicative of an arterial blood pressure waveform in the arterial line 1.

The method further comprises a step 120 of determining a hemodynamic contraction parameter related to a contraction of the heart based on the hemodynamic signal and a step 130 of determining a hemodynamic relaxation parameter related to a relaxation of the heart based on the hemodynamic signal provided in step 110.

In a step 140, a clotting event in the arterial line 1 is determined based on the determined hemodynamic contraction parameter and the hemodynamic relaxation parameter in case the hemodynamic contraction parameter indicates a decrease of contractility and the hemodynamic relaxation parameter indicates an increase of contractility.

After determining a clotting event in step 140, a test to confirm that the arterial line is clotted can be executed in a step 145. Such a test can, for instance, include an automatic or request for a manual fast flush test. The test in step 145 preferentially determines arterial line characteristics such as natural frequency and damping by a tapping the catheter of fast flush to verify the presence of a clotting event.

After determining a clotting event in step 140, and optionally under the precondition that the test in step 145 confirmed the presence of the clotting event, a signal notifying of the determined clotting event is provided in a step 150.

Next, in an optional step 160 a flushing event of the arterial line 1 is executed, for instance as a result to an automated flushing, recognized, for instance as a result of a manual flushing, or recommended, for instance using a signal being notified to an operator.

Subsequently, in a step 170, whether a change in at least one predetermined hemodynamic parameter before and after the flushing event exceeds a predetermined flushing threshold is used to verify the occurrence of a clotting event.

The method further comprises a step 180 of selecting, out of a list of available hemodynamic parameters, a subset of the available hemodynamic parameters as individual clotting parameters. The individual clotting parameters can be used to determine subsequent clotting events, wherein preferentially machine learning prediction algorithms are employed to adapt the hemodynamic contraction parameter and/or the hemodynamic relaxation parameter based on the individual clotting parameters.

The method further includes a step 190 of adjusting at least one of the increase threshold and the decrease threshold using the hemodynamic contraction parameter and the hemodynamic relaxation parameter before and after flushing the arterial line 1. In this example, the increase threshold and the decrease threshold are used to describe an increase of contractility and a decrease of contractility, respectively.

Finally, the method returns preferentially to step 110.

It should be noted that the repeated or continuous operation of the method is particularly beneficial during monitoring of a patient. In other examples, the repeated execution of the method is not necessary, for instance, when offline data is analysed. The offline analysis of data is particularly advantageous in case the hemodynamic contraction parameter and the hemodynamic relaxation parameter are to be defined and/or optimized.

Further, it should be noted that steps 110, 120, 130, and 140 are essential to the present disclosure, while the further steps 145, 150, 160, 170, 180 and 190 are optional steps which describe preferred embodiments allowing to obtain further advantages over the essential method steps 110 to 140.

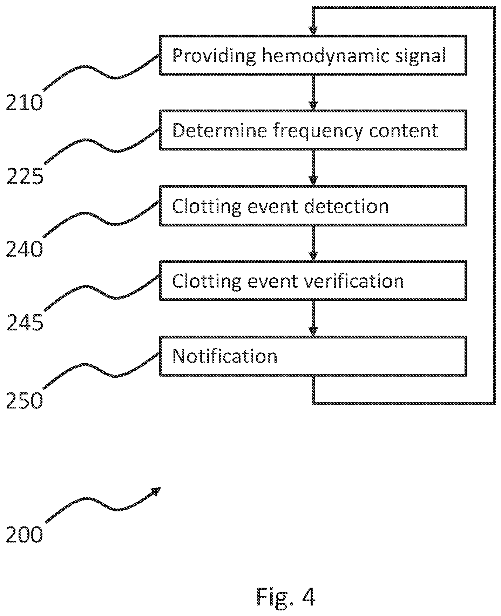

Further, the steps showing a certain order in Fig. 3, and also in Fig. 4 described below, does not imply that they are executed subsequently, i.e., no restrictions with regard to their execution time are implied. For instance, steps 120 and 130 can be executed at the same time and, preferentially, in substance simultaneously with the provided hemodynamic signal in step 110.

Fig. 4 schematically and exemplarily illustrates a further method 200 for notifying a clotting event in an arterial line. The method 200 can be combined with the method 100 illustrated in Fig. 3 and in particular steps described with reference to method 100 can be integrated into the method 200.

The method comprises a step 210 of providing a hemodynamic signal indicative of an arterial blood pressure waveform in the arterial line 1.

The method further comprises a step 225 of determining a frequency content based on the hemodynamic signal provided in step 110.

In a step 240, a clotting event in the arterial line 1 is determined based on the determined frequency content of the hemodynamic signal, wherein in particular the clotting event is determined in case the frequency content or a change of the frequency content exceeds a predetermined threshold.

After determining a clotting event in step 240, a test to confirm that the arterial line is clotted can be executed in a step 245. Such a test can, similar to step 145, include for instance an automatic or request for a manual fast flush test. The test in step 245 preferentially determines arterial line characteristics such as natural frequency and damping by a tapping the catheter of fast flush to verify the presence of a clotting event.

After determining a clotting event in step 240, and optionally under the precondition that the test in step 245 confirmed the presence of the clotting event, a signal notifying of the determined clotting event is provided in a step 250.

Finally, the method returns preferentially to step 210.

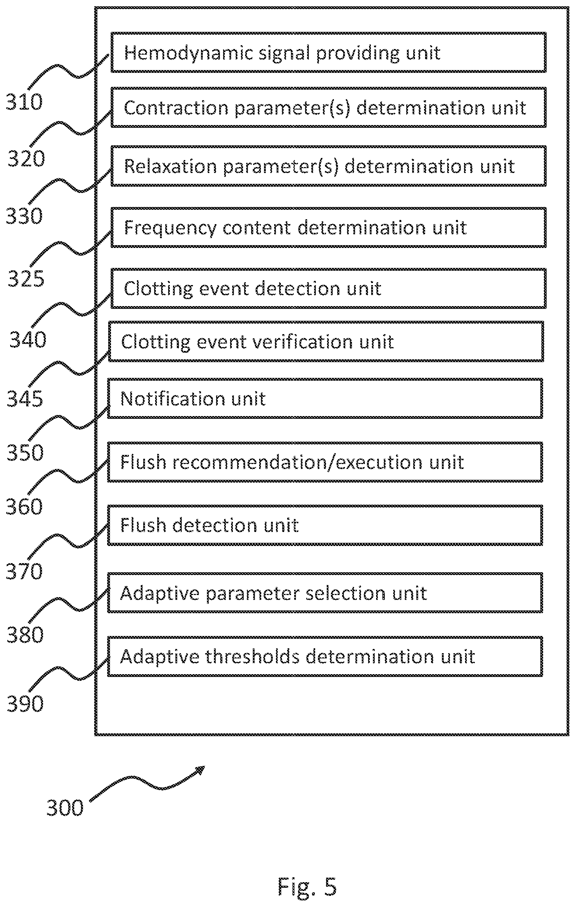

Fig. 5 schematically and exemplarily illustrates a system 300 for notifying a clotting event in an arterial line, such as arterial line 1 of human being 2 illustrated in Fig. 1.

The system 300 comprises a hemodynamic signal providing unit 310 for providing a hemodynamic signal indicative of an arterial blood pressure waveform 10 in the arterial line 1.

The system 300 may further comprise a hemodynamic contraction parameter determination unit 320 for determining a hemodynamic contraction parameter related to a contraction of the heart based on the hemodynamic signal provided by hemodynamic signal providing unit 310.

The system 300 may further comprise a hemodynamic relaxation parameter determination unit 330 for determining a hemodynamic relaxation parameter related to a relaxation of the heart based on the hemodynamic signal provided by hemodynamic signal providing unit 310.

The system 300 may further comprise a frequency content determination unit 325 for determining a frequency content of the hemodynamic signal provided by hemodynamic signal providing unit 310.

The system 300 further comprises a clotting event determination unit 340 for determining a clotting event in the arterial line 1. The clotting event determination unit 340 may be configured to determine the clotting event in case the hemodynamic contraction parameter indicates a decrease of contractility and the hemodynamic relaxation parameter indicates an increase of contractility. Alternatively or additionally, the clotting event determination unit may be configured to determine a clotting event in the arterial line 1 based on the frequency content of the hemodynamic signal, wherein in particular the clotting event is determined in case the frequency content or a change of the frequency content exceeds a predetermined threshold.

The system 300 may further comprise a clotting event verification unit 345. After determining a clotting event by clotting event detection unit 340, a test to confirm that the arterial line is clotted can be executed according to step 145 of method 100 or step 245 of method 200.

The system 300 further comprises a notification unit 350 for providing a signal notifying of the determined clotting event. The notification unit 345 may be operably coupled to the display 8 of patient monitor 6 to display the notification thereon. In other examples, other forms of acoustical, optical or data signal notifications can be implemented.

The system 300 may further comprise a flush recommendation/execution unit 360 configured to execute step 160 of method 100. The system 300 may further comprise a flush detection unit 370 configured to execute step 170 of method 100. The system 300 may further comprise an adaptive parameter selection unit 380 configured to execute step 180 of method 100. Finally, the system 300 may further comprise an adaptive thresholds determination unit 390 configured to execute step 190 of method 100.

By simultaneously comparing the waveforms obtained with a non-invasive continuous blood pressure technology such as the ClearSight system by Edwards Lifesciences of Irvine, CA, with the radial artery pressure waveform characteristic patterns in the development of clotting in an arterial line can be recognized in the signals from the radial catheter.

It can be verified that the periods of clotting detected by the method 100 of the present disclosure were eventually but not always also detected by the anesthesiologist (experimenter), since a flush of the catheter manometer system should usually follow such an event. Usually the invasive arterial blood pressure measurement improves significantly after a flush of the catheter system. Therefore, a jump in hemodynamic parameters before and after the flush of the arterial system to solve the clotting, can be used to confirm that the event was indeed due to clotting.

The disclosure originates from the simultaneous (offline) comparison of ClearSight and radial blood pressure waveform in available clinical data. From such observations the inventors were able to derive a pattern that can be recognized also when monitoring only the arterial line without another blood pressure for comparison.

With the here described method a typical clotting event in an arterial line can be recognized. This event can be used, for instance, to warn the anesthesiologist that radial artery data are unreliable due to clotting. The catheter system has to be flushed to solve the issue.

The various illustrative logical blocks and units described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm and the units of the system described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, non-transitory computer readable medium, or any other form of storage medium known in the art. An exemplary storage medium is coupled to the processor such the processor can read information from, and write information to, the storage medium. In the alternative, the storage medium may be integral to the processor.

## Claims

1. A system for notifying a clotting event in an arterial line (1) of a human being (2), the system comprising:
- a hemodynamic signal providing unit (310) for providing (110) a hemodynamic signal indicative of an arterial blood pressure waveform (10) in the arterial line (1),
- a hemodynamic contraction parameter determination unit (320) for determining (120) a hemodynamic contraction parameter related to a contraction of the heart based on the hemodynamic signal,
- a hemodynamic relaxation parameter determination unit (330) for determining (130) a hemodynamic relaxation parameter related to a relaxation of the heart based on the hemodynamic signal,
- a clotting event determination unit (340) for determining (140) a clotting event in the arterial line in case the hemodynamic contraction parameter indicates a decrease of contractility and the hemodynamic relaxation parameter indicates an increase of contractility, and
- a notification unit (345) for providing (150) a signal notifying of the determined clotting event.

2. The system for notifying a clotting event in an arterial line (1) of a human being (2) according to claim 1, wherein:
a) the hemodynamic contraction parameter includes at least one hemodynamic parameter derivable from the arterial blood pressure waveform between a begin of the systolic phase and a closure of the aortic valve at the end of systole, wherein the hemodynamic contraction parameter in particular includes at least one of:
- a maximum change in blood pressure during systole,
- an end systolic pressure,
- an area under the arterial blood pressure waveform curve above end diastolic pressure between the begin of the systolic phase and the closure of the aortic valve at the end of systole; and/or
b) the hemodynamic relaxation parameter includes at least one of:
- an end-diastolic pressure,
- a mean-arterial pressure,
- an interbeat interval.

3. The system for notifying a clotting event in an arterial line (1) of a human being (2) according to any of the preceding claims, wherein at least one of the hemodynamic contraction parameter determination unit (320) and the hemodynamic relaxation parameter determination unit (330) is further configured to determine the respective hemodynamic contraction parameter or the hemodynamic relaxation parameter as an average over a predefined period of time, in particular as a 20 second average.

4. The system for notifying a clotting event in an arterial line (1) of a human being (2) according to claim 3, wherein the at least one of the hemodynamic contraction parameter determination unit (320) and the hemodynamic relaxation parameter determination unit (330) is further configured so that the determination as an average includes at least one of a filtering process and determining a moving average.

5. The system for notifying a clotting event in an arterial line (1) of a human being (2) according to any of the preceding claims, wherein the system further comprises an indication unit configured to indicate a decrease or increase of the hemodynamic contraction parameter and the hemodynamic relaxation parameter based on a comparison of two subsequent values of the hemodynamic contraction parameter and the hemodynamic relaxation parameter, respectively.

6. The system for notifying a clotting event in an arterial line (1) of a human being (2) according to any of the preceding claims, wherein the system further comprises a flush recommendation/execution unit (360) configured to recommend a flushing event of the arterial line and/or a flush detection unit (370) configured to recognize a flushing event of the arterial line and to verify (170) a clotting event in case a change in at least one predetermined hemodynamic parameter, in particular of maximum change in blood pressure during systole or end systolic pressure, before and after the flushing event exceeds a predetermined flushing threshold, wherein the flush detection unit (370), in particular, is further configured to determine the flushing event of the arterial line based on a significant change in at least one of maximum pressure gradient and systolic pressure.

7. The system for notifying a clotting event in an arterial line (1) of a human being (2) according to claim 6, wherein the system further comprises a adaptive parameter selection unit (380) configured to select (180), out of a list of available hemodynamic parameters, a subset of the available hemodynamic parameters as individual clotting parameters, wherein a hemodynamic parameter classifies as individual clotting parameter in case a change between before and after the flushing event exceeds a predefined threshold.

8. The system for notifying a clotting event in an arterial line (1) of a human being (2) according to claim 7, wherein, for determining a subsequent clotting event, at least one of the hemodynamic contraction parameter and the hemodynamic relaxation parameter includes a hemodynamic parameter included in the individual clotting parameters.

9. The system for notifying a clotting event in an arterial line (1) of a human being (2) according to any of claims 6 to 8, wherein the system is configured to adapt at least one of the hemodynamic contraction parameter and the hemodynamic relaxation parameter using a machine learning prediction algorithm.

10. The system for notifying a clotting event in an arterial line (1) of a human being (2) according to any of the preceding claims, configured to indicate based on the hemodynamic contraction parameter a decrease of contractility in case a change in the hemodynamic contraction parameter exceeds a decrease threshold and wherein the system is further configured to indicate based on the hemodynamic relaxation parameter indicates an increase of contractility in case a change in the hemodynamic relaxation parameter exceeds an increase threshold, wherein the system is further configured to adjust at least one of the increase threshold and the decrease threshold based on previously determined clotting events, in particular based on past verified clotting events,
optionally wherein the system further comprises an adaptive thresholds determination unit (390) configured to adjust (190) at least one of the increase threshold and the decrease threshold using the hemodynamic contraction parameter and the hemodynamic relaxation parameter before and after flushing the arterial line, respectively.

11. The system for notifying a clotting event in an arterial line (1) of a human being (2) according to claim 10, configured to adapt prediction parameters including the increase threshold and the decrease threshold using a machine learning prediction algorithm.

12. The system for notifying a clotting event in an arterial line (1) of a human being (2) according to any of the preceding claims, wherein the system further comprises a frequency content determination unit (325) configured to determine a frequency content of the provided hemodynamic signal wherein the clotting event determination unit (340) is further configured to determine the clotting event additionally based on the determined frequency content, wherein in particular the clotting event is determined in case the frequency content or a change of the frequency content exceeds a predetermined threshold.

13. The system for notifying a clotting event in an arterial line (1) of a human being (2) of claim 12, wherein the frequency content determination unit (325) is further configured to determine the frequency content as a sum of the frequency contents within a frequency range of interest, the frequency range of interest including a frequency of interest and in particular a resonance such as a natural frequency of the arterial line (1), wherein the frequency content is particularly preferably determined as a relative value scaled with a sum of the frequency contents over all frequencies,
optionally further configured to determine the frequency range of interest relative to the frequency of interest and in particular includes frequency within 50% to 200% of the frequency of interest and/or includes frequencies with amplitudes above 50% of the amplitude of the frequency of interest.

14. The system for notifying a clotting event in an arterial line (1) of a human being (2) according to any of the preceding claims, wherein the system is further configured to execute, after determining a clotting event, a flush test, the flush test comprising an opening of a pressurised reservoir and a sudden stop of the flow resulting in a sudden change of blood pressure, and wherein the system is further configured to evaluate oscillations of the arterial line system based on the provided hemodynamic signal indicative of the arterial blood pressure waveform for a period following the sudden stop, the oscillations in the hemodynamic signal being induced by the sudden change of blood pressure, and wherein the system is further configured to assess damping and natural frequency of the arterial line system based on the evaluated oscillations,
optionally wherein the flush test includes at least one of:
- an automated fast flush, in which the opening of the pressurized reservoir and/or the sudden stop of the flow is automatically operated using an automated flushing means capable of inducing a flush which can be a time wise short injection of a liquid, preferably a saline solution, with pressure into the arterial line,
- a manual fast flush, in which an instruction to perform the manual opening of the pressurized reservoir and/or the sudden stop of the flow is signalled to an operator, and
- a manual tapping test, wherein the tapping test comprises increasing once or several times the blood pressure by applying an increased pressure through the arterial line, for instance by tapping on flexible tubing connected to the arterial line, preferentially with a sudden release of the applied pressure, wherein the manual tapping test includes signalling an instruction to perform the manual tapping test to the operator.

15. The system for notifying a clotting event in an arterial line (1) of a human being (2) according to claim 14, wherein the system is further configured to, after determining a clotting event, verify that the determined clotting event is indeed a true clotting event based on the assessed damping and the natural frequency of the arterial system, in particular, wherein the system is further configured to, in case of a verified true clotting event, to execute an automated flushing wherein the arterial line comprises an automated flushing means capable of inducing a flush which can be a time wise short injection of a liquid, preferably a saline solution, with pressure into the arterial line in order to free the arterial line from clotted blood.

## Patentansprüche

1. System zum Benachrichtigen über ein Gerinnungsereignis in einer arteriellen Leitung (1) eines Menschen (2), wobei das System Folgendes umfasst:
- eine hämodynamische Signalbereitstellungseinheit (310) zum Bereitstellen (110) eines hämodynamischen Signals, das eine arterielle Blutdruckwellenform (10) in der arteriellen Leitung (1) angibt,
- eine hämodynamische Kontraktionsparameterbestimmungseinheit (320) zum Bestimmen (120) eines hämodynamischen Kontraktionsparameters, der mit einer Kontraktion des Herzens in Beziehung steht, basierend auf dem hämodynamischen Signal,
- eine hämodynamische Relaxationsparameterbestimmungseinheit (330) zum Bestimmen (130) eines hämodynamischen Relaxationsparameters, der mit einer Relaxation des Herzens in Beziehung steht, basierend auf dem hämodynamischen Signal,
- eine Gerinnungsereignisbestimmungseinheit (340) zum Bestimmen (140) eines Gerinnungsereignisses in der arteriellen Leitung, falls der hämodynamische Kontraktionsparameter eine Abnahme der Kontraktilität angibt und der hämodynamische Relaxationsparameter eine Zunahme der Kontraktilität angibt, und
- eine Benachrichtigungseinheit (345) zum Bereitstellen (150) eines Signals, welches über das bestimmte Gerinnungsereignis benachrichtigt.

2. System zum Benachrichtigen über ein Gerinnungsereignis in einer arteriellen Leitung (1) eines Menschen (2) nach Anspruch 1, wobei:
a) der hämodynamische Kontraktionsparameter mindestens einen hämodynamischen Parameter einschließt, der von der arteriellen Blutdruckwellenform zwischen einem Beginn der systolischen Phase und einem Schließen der Aortenklappe am Ende der Systole ableitbar ist, wobei der hämodynamische Kontraktionsparameter insbesondere mindestens eines von Folgendem einschließt:
- eine maximale Änderung des Blutdrucks während der Systole,
- einen systolischen Enddruck,
- eine Fläche unter der Kurve der arteriellen Blutdruckwellenform oberhalb des diastolischen Enddrucks zwischen dem Beginn der systolischen Phase und dem Schließen der Aortenklappe am Ende der Systole; und/oder
b) der hämodynamische Relaxationsparameter mindestens eines von Folgendem einschließt:
- einem enddiastolischen Druck,
- einem mittleren arteriellen Druck,
- einem Interbeat-Intervall.

3. System zum Benachrichtigen über ein Gerinnungsereignis in einer arteriellen Leitung (1) eines Menschen (2) nach einem der vorhergehenden Ansprüche, wobei mindestens eine von der hämodynamischen Kontraktionsparameterbestimmungseinheit (320) und der hämodynamischen Relaxationsparameterbestimmungseinheit (330) ferner dazu ausgelegt ist, den jeweiligen hämodynamischen Kontraktionsparameter oder den hämodynamischen Relaxationsparameter als einen Mittelwert über einen vordefinierten Zeitraum, insbesondere als einen Mittelwert von 20 Sekunden, zu bestimmen.

4. System zum Benachrichtigen über ein Gerinnungsereignis in einer arteriellen Leitung (1) eines Menschen (2) nach Anspruch 3, wobei die mindestens eine von der hämodynamischen Kontraktionsparameterbestimmungseinheit (320) und der hämodynamischen Relaxationsparameterbestimmungseinheit (330) ferner so ausgelegt ist, dass die Bestimmung als Mittelwert mindestens eines von einem Filterungsprozess und Bestimmen eines gleitenden Mittelwerts einschließt.

5. System zum Benachrichtigen über ein Gerinnungsereignis in einer arteriellen Leitung (1) eines Menschen (2) nach einem der vorhergehenden Ansprüche, wobei das System ferner eine Angabeeinheit umfasst, die dazu ausgelegt ist, eine Abnahme oder Zunahme des hämodynamischen Kontraktionsparameters und des hämodynamischen Relaxationsparameters basierend auf einem Vergleich von zwei nachfolgenden Werten des hämodynamischen Kontraktionsparameters bzw. des hämodynamischen Relaxationsparameters anzugeben.

6. System zum Benachrichtigen über ein Gerinnungsereignis in einer arteriellen Leitung (1) eines Menschen (2) nach einem der vorhergehenden Ansprüche, wobei das System ferner eine Spülempfehlungs-/- ausführungseinheit (360), die dazu ausgelegt ist, ein Spülereignis der arteriellen Leitung zu empfehlen, und/oder eine Spüldetektionseinheit (370) umfasst, die dazu ausgelegt ist, ein Spülereignis der arteriellen Leitung zu erkennen und ein Gerinnungsereignis zu verifizieren (170), falls eine Änderung des mindestens einen vorbestimmten hämodynamischen Parameters, insbesondere einer maximalen Änderung des Blutdrucks während der Systole oder des systolischen Enddrucks, vor und nach dem Spülereignis eine vorbestimmte Spülschwelle überschreitet, wobei die Spüldetektionseinheit (370) insbesondere ferner dazu ausgelegt ist, das Spülereignis der arterielle Leitung basierend auf einer signifikanten Änderung in mindestens einem von maximalem Druckgradienten und systolischem Drucks zu bestimmen.

7. System zum Benachrichtigen über ein Gerinnungsereignis in einer arteriellen Leitung (1) eines Menschen (2) nach Anspruch 6, wobei das System ferner eine adaptive Parameterauswahleinheit (380) umfasst, die dazu ausgelegt ist, aus einer Liste verfügbarer hämodynamischer Parameter eine Teilmenge der verfügbaren hämodynamischen Parameter als individuelle Gerinnungsparameter auszuwählen (180), wobei ein hämodynamischer Parameter als individueller Gerinnungsparameter klassifiziert wird, falls eine Änderung zwischen vor und nach dem Spülereignis eine vordefinierte Schwelle überschreitet.

8. System zum Benachrichtigen über ein Gerinnungsereignis in einer arteriellen Leitung (1) eines Menschen (2) nach Anspruch 7, wobei zum Bestimmen eines nachfolgenden Gerinnungsereignisses mindestens einer von dem hämodynamischen Kontraktionsparameter und dem hämodynamischen Relaxationsparameter einen hämodynamischen Parameter einschließt, der in den individuellen Gerinnungsparametern eingeschlossen ist.

9. System zum Benachrichtigen über ein Gerinnungsereignis in einer arteriellen Leitung (1) eines Menschen (2) nach einem der Ansprüche 6 bis 8, wobei das System dazu ausgelegt ist, mindestens einen von dem hämodynamischen Kontraktionsparameter und dem hämodynamischen Relaxationsparameter unter Verwendung eines Maschinenlernvorhersagealgorithmus anzupassen.

10. System zum Benachrichtigen über ein Gerinnungsereignis in einer arteriellen Leitung (1) eines Menschen (2) nach einem der vorhergehenden Ansprüche, das ausgelegt ist zum Angeben einer Abnahme der Kontraktilität basierend auf dem hämodynamischen Kontraktionsparameter, falls eine Änderung des hämodynamischen Kontraktionsparameters eine Abnahmeschwelle überschreitet, und wobei das System ferner ausgelegt ist zum Angeben einer Zunahme der Kontraktilität basierend auf dem hämodynamischen Relaxationsparameter, falls eine Änderung des hämodynamischen Relaxationsparameters eine Zunahmeschwelle überschreitet, wobei das System ferner dazu ausgelegt ist, mindestens eine von der Zunahmeschwelle und der Abnahmeschwelle basierend auf zuvor bestimmten Gerinnungsereignissen, insbesondere basierend auf vergangenen verifizierten Gerinnungsereignissen, anzupassen,
wobei das System gegebenenfalls ferner eine adaptive Schwellenbestimmungseinheit (390) umfasst, die dazu ausgelegt ist, mindestens eine von der Zunahmeschwelle und der Abnahmeschwelle unter Verwendung des hämodynamischen Kontraktionsparameters und des hämodynamischen Relaxationsparameters vor bzw. nach dem Spülen der arteriellen Leitung anzupassen (190).

11. System zum Benachrichtigen über ein Gerinnungsereignis in einer arteriellen Leitung (1) eines Menschen (2) nach Anspruch 10, das ausgelegt ist zum Anpassen von Prädiktionsparametern einschließlich der Zunahmeschwelle und der Abnahmeschwelle unter Verwendung eines Maschinenlernprädiktionsalgorithmus.

12. System zum Benachrichtigen über ein Gerinnungsereignis in einer arteriellen Leitung (1) eines Menschen (2) nach einem der vorhergehenden Ansprüche, wobei das System ferner eine Frequenzinhaltsbestimmungseinheit (325) umfasst, die dazu ausgelegt ist, einen Frequenzinhalt des bereitgestellten hämodynamischen Signals zu bestimmen, wobei die Gerinnungsereignisbestimmungseinheit (340) ferner dazu ausgelegt ist, das Gerinnungsereignis zusätzlich basierend auf dem bestimmten Frequenzinhalt zu bestimmen, wobei insbesondere das Gerinnungsereignis bestimmt wird, falls der Frequenzinhalt oder eine Änderung des Frequenzinhalts eine vorbestimmte Schwelle überschreitet.

13. System zum Benachrichtigen über ein Gerinnungsereignis in einer arteriellen Leitung (1) eines Menschen (2) nach Anspruch 12, wobei die Frequenzinhaltsbestimmungseinheit (325) ferner dazu ausgelegt ist, den Frequenzinhalt als eine Summe der Frequenzinhalte innerhalb eines interessierenden Frequenzbereichs zu bestimmen, wobei der interessierende Frequenzbereich eine interessierende Frequenz und insbesondere eine Resonanz, wie eine Eigenfrequenz der arteriellen Leitung (1), einschließt, wobei der Frequenzgehalt besonders bevorzugt als ein relativer Wert bestimmt wird, der mit einer Summe der Frequenzinhalte über alle Frequenzen skaliert wird,
ferner gegebenenfalls dazu ausgelegt, den interessierenden Frequenzbereich relativ zu der interessierenden Frequenz zu bestimmen, und insbesondere einschließlich einer Frequenz innerhalb von 50 % bis 200 % der interessierenden Frequenz und/oder Frequenzen mit Amplituden oberhalb 50 % der Amplitude der interessierenden Frequenz.

14. System zum Benachrichtigen über ein Gerinnungsereignis in einer arteriellen Leitung (1) eines Menschen (2) nach einem der vorhergehenden Ansprüche, wobei das System ferner dazu ausgelegt ist, nach Bestimmen eines Gerinnungsereignisses einen Spültest auszuführen, wobei der Spültest eine Öffnung eines druckbeaufschlagten Reservoirs und ein plötzliches Stoppen des Flusses umfasst, was zu einer plötzlichen Änderung des Blutdrucks führt, und wobei das System ferner dazu ausgelegt ist, Oszillationen des arteriellen Leitungssystems basierend auf dem bereitgestellten hämodynamischen Signal, das die arterielle Blutdruckwellenform angibt, für einen Zeitraum nach dem plötzlichen Stopp zu evaluieren, wobei die Oszillationen in dem hämodynamischen Signal durch die plötzliche Änderung des Blutdrucks induziert werden, und wobei das System ferner dazu ausgelegt ist, Dämpfung und Eigenfrequenz des arteriellen Leitungssystems basierend auf den evaluierten Oszillationen zu bewerten,
wobei der Spültest gegebenenfalls mindestens eines von Folgendem einschließt:
- eine automatisierte Schnellspülung, bei der die Öffnung des druckbeaufschlagten Reservoirs und/oder der plötzliche Stopp des Flusses automatisch mit Hilfe eines automatisierten Spülmittels betrieben wird, das eine Spülung induzieren kann, die eine zeitweise kurze Injektion einer Flüssigkeit, vorzugsweise einer Salzlösung, mit Druck in die arterielle Leitung sein kann,
- eine manuelle Schnellspülung, bei der eine Anweisung zum Durchführen des manuellen Öffnens des druckbeaufschlagten Reservoirs und/oder zum plötzlichen Stoppen des Flusses an einen Bediener signalisiert wird, und
- einen manuellen Klopftest, wobei der Klopftest ein-oder mehrmaliges Erhöhen des Blutdrucks durch Anlegen eines erhöhten Drucks durch die arterielle Leitung umfasst, beispielsweise durch Klopfen auf flexible, mit der arterielle Leitung verbundene Schläuche, vorzugsweise mit einem plötzlichen Entspannen des angelegten Drucks, wobei der manuelle Klopftest Signalisieren einer Anweisung zur Durchführung des manuellen Klopftests an den Bediener umfasst.

15. System zum Benachrichtigen über ein Gerinnungsereignis in einer arteriellen Leitung (1) eines Menschen (2) nach Anspruch 14, wobei das System ferner dazu ausgelegt ist, nach Bestimmen eines Gerinnungsereignisses zu verifizieren, dass das bestimmte Gerinnungsereignis tatsächlich ein wahres Gerinnungsereignis ist, basierend auf der bewerteten Dämpfung und der Eigenfrequenz des arteriellen Systems, wobei insbesondere das System ferner dazu ausgelegt ist, im Fall eines verifizierten wahren Gerinnungsereignisses eine automatisierte Spülung durchzuführen, bei der die arterielle Leitung ein automatisiertes Spülmittel umfasst, das eine Spülung induzieren kann, die eine zeitlich kurze Injektion einer Flüssigkeit, vorzugsweise einer Kochsalzlösung, mit Druck in die arterielle Leitung sein kann, um die arterielle Leitung von geronnenem Blut zu befreien.

## Revendications

1. Système de notification d'un événement de coagulation dans une ligne artérielle (1) d'un être humain (2), le système comprenant :
- une unité de fourniture d'un signal hémodynamique (310) pour la fourniture (110) d'un signal hémodynamique indicatif d'une forme d'onde de pression sanguine artérielle (10) dans la ligne artérielle (1),
- une unité de détermination de paramètre de contraction hémodynamique (320) pour la détermination (120) d'un paramètre de contraction hémodynamique lié à une contraction du cœur sur la base du signal hémodynamique,
- une unité de détermination d'un paramètre de relaxation hémodynamique (330) pour la détermination (130) d'un paramètre de relaxation hémodynamique lié à une relaxation du cœur sur la base du signal hémodynamique,
- une unité de détermination d'un événement de coagulation (340) pour la détermination (140) d'un événement de coagulation dans la ligne artérielle dans le cas où le paramètre de contraction hémodynamique indique une diminution de contractilité et le paramètre de relaxation hémodynamique indique une augmentation de contractilité, et
- une unité de notification (345) pour la fourniture (150) d'un signal notifiant de l'événement de coagulation déterminé.

2. Système de notification d'un événement de coagulation dans une ligne artérielle (1) d'un être humain (2) selon la revendication 1,
a) le paramètre de contraction hémodynamique comprenant au moins un paramètre hémodynamique dérivable de la forme d'onde de pression sanguine artérielle entre un début de la phase systolique et une fermeture de la valve aortique en fin de systole, le paramètre de contraction hémodynamique comprenant en particulier au moins l'un parmi :
- une variation maximale de la pression artérielle pendant la systole,
- une pression systolique de fin,
- une aire sous la courbe de forme d'onde de pression sanguine artérielle supérieure à la pression diastolique de fin entre le début de la phase systolique et la fermeture de la valve aortique en fin de systole ; et/ou
b) le paramètre de relaxation hémodynamique comprenant au moins l'un parmi :
- une pression diastolique de fin,
- une pression artérielle moyenne,
- un intervalle interbattement.

3. Système de notification d'un événement de coagulation dans une ligne artérielle (1) d'un être humain (2) selon l'une quelconque des revendications précédentes, au moins l'une parmi l'unité de détermination de paramètre de contraction hémodynamique (320) et l'unité de détermination de paramètre de relaxation hémodynamique (330) étant en outre configurée pour déterminer le paramètre de contraction hémodynamique respectif ou le paramètre de relaxation hémodynamique en tant que moyenne sur une période de temps prédéfinie, en particulier en tant que moyenne sur 20 secondes.

4. Système de notification d'un événement de coagulation dans une ligne artérielle (1) d'un être humain (2) selon la revendication 3, l'au moins une unité parmi l'unité de détermination de paramètre de contraction hémodynamique (320) et l'unité de détermination de paramètre de relaxation hémodynamique (330) étant en outre configurée de sorte que la détermination en tant que moyenne inclut au moins l'un d'un processus de filtrage et de la détermination d'une moyenne mobile.

5. Système de notification d'un événement de coagulation dans une ligne artérielle (1) d'un être humain (2) selon l'une quelconque des revendications précédentes, le système comprenant en outre une unité d'indication configurée pour indiquer une diminution ou une augmentation du paramètre de contraction hémodynamique et du paramètre de relaxation hémodynamique sur la base d'une comparaison de deux valeurs ultérieures du paramètre de contraction hémodynamique et du paramètre de relaxation hémodynamique, respectivement.

6. Système de notification d'un événement de coagulation dans une ligne artérielle (1) d'un être humain (2) selon l'une quelconque des revendications précédentes, le système comprenant en outre une unité de recommandation/exécution de rinçage (360) configurée pour recommander un événement de rinçage de la ligne artérielle et/ou une unité de détection de rinçage (370) configurée pour reconnaître un événement de rinçage de la ligne artérielle et pour vérifier (170) un événement de coagulation en cas de changement d'au moins un paramètre hémodynamique prédéterminé, en particulier, une variation maximale de la pression sanguine pendant la systole ou la pression systolique de fin, entre avant et après que l'événement de rinçage dépasse un seuil de rinçage prédéterminé, l'unité de détection de rinçage (370), en particulier, étant en outre configurée pour déterminer l'événement de rinçage de la ligne artérielle sur la base d'une variation significative d'au moins l'un d'un gradient de pression maximal et d'une pression systolique.

7. Système de notification d'un événement de coagulation dans une ligne artérielle (1) d'un être humain (2) selon la revendication 6, le système comprenant en outre une unité de sélection de paramètres adaptatifs (380) configurée pour sélectionner (180), parmi une liste de paramètres hémodynamiques disponibles, un sous-ensemble des paramètres hémodynamiques disponibles en tant que paramètres de coagulation individuels, un paramètre hémodynamique se classant en tant que paramètre de coagulation individuel au cas où un changement entre avant et après l'événement de rinçage dépasse un seuil prédéfini.

8. Système de notification d'un événement de coagulation dans une ligne artérielle (1) d'un être humain (2) selon la revendication 7, pour déterminer un événement de coagulation ultérieur, au moins l'un du paramètre de contraction hémodynamique et du paramètre de relaxation hémodynamique comprenant un paramètre hémodynamique inclus dans les paramètres de coagulation individuels.

9. Système de notification d'un événement de coagulation dans une ligne artérielle (1) d'un être humain (2) selon l'une quelconque des revendications 6 à 8, le système étant configuré pour adapter au moins l'un du paramètre de contraction hémodynamique et du paramètre de relaxation hémodynamique en utilisant un algorithme de prédiction par apprentissage automatique.

10. Système de notification d'un événement de coagulation dans une ligne artérielle (1) d'un être humain (2) selon l'une quelconque des revendications précédentes, configuré pour indiquer, sur la base du paramètre de contraction hémodynamique, une diminution de la contractilité dans le cas où un changement du paramètre de contraction hémodynamique dépasse un seuil de diminution, et le système étant en outre configuré pour indiquer, sur la base du paramètre de relaxation hémodynamique, une augmentation de la contractilité dans le cas où un changement du paramètre de relaxation hémodynamique dépasse un seuil d'augmentation, le système étant en outre configuré pour ajuster au moins l'un du seuil d'augmentation et du seuil de diminution sur la base d'événements de coagulation précédemment déterminés, en particulier sur la base d'événements de coagulation vérifiés antérieurement,
éventuellement, le système comprenant en outre une unité de détermination de seuils adaptatifs (390) configurée pour ajuster (190) au moins l'un du seuil d'augmentation et du seuil de diminution au moyen du paramètre de contraction hémodynamique et du paramètre de relaxation hémodynamique avant et après rinçage de la ligne artérielle, respectivement.

11. Système de notification d'un événement de coagulation dans une ligne artérielle (1) d'un être humain (2) selon la revendication 10, configuré pour adapter des paramètres de prédiction comprenant le seuil d'augmentation et le seuil de diminution en utilisant un algorithme de prédiction par apprentissage automatique.

12. Système de notification d'un événement de coagulation dans une ligne artérielle (1) d'un être humain (2) selon l'une quelconque des revendications précédentes, le système comprenant en outre une unité de détermination de contenu fréquentiel (325) configurée pour déterminer un contenu fréquentiel du signal hémodynamique fourni, l'unité de détermination d'événement de coagulation (340) étant en outre configurée pour déterminer l'événement de coagulation en outre en fonction du contenu fréquentiel déterminé, l'événement de coagulation étant en particulier déterminé dans le cas où le contenu fréquentiel ou un changement du contenu fréquentiel dépasse un seuil prédéterminé.

13. Système de notification d'un événement de coagulation dans une ligne artérielle (1) d'un être humain (2) selon la revendication 12, l'unité de détermination de contenu fréquentiel (325) étant en outre configurée pour déterminer le contenu fréquentiel sous la forme d'une somme des contenus fréquentiels dans une plage de fréquences d'intérêt, la plage de fréquences d'intérêt comprenant une fréquence d'intérêt et en particulier une résonance telle qu'une fréquence naturelle de la ligne artérielle (1), le contenu fréquentiel étant de manière particulièrement préférée déterminé comme une valeur relative mise à l'échelle avec une somme des contenus fréquentiels sur toutes les fréquences,
éventuellement, configuré en outre pour déterminer la plage de fréquences d'intérêt par rapport à la fréquence d'intérêt et comportant en particulier une fréquence comprise entre 50 % et 200 % de la fréquence d'intérêt et/ou comportant des fréquences d'amplitudes supérieures à 50 % de l'amplitude de la fréquence d'intérêt.

14. Système de notification d'un événement de coagulation dans une ligne artérielle (1) d'un être humain (2) selon l'une quelconque des revendications précédentes, le système étant en outre configuré pour exécuter, après détermination d'un événement de coagulation, un test de rinçage, le test de rinçage comprenant une ouverture d'un réservoir pressurisé et un arrêt soudain de l'écoulement résultant en un changement soudain de la pression sanguine, et le système étant en outre configuré pour évaluer les oscillations du système de ligne artérielle sur la base du signal hémodynamique fourni indicatif de la forme d'onde de pression sanguine artérielle pendant une période suivant l'arrêt brusque, les oscillations du signal hémodynamique étant induites par le changement soudain de la pression artérielle, et le système étant en outre configuré pour évaluer l'amortissement et la fréquence naturelle du système de ligne artérielle sur la base des oscillations évaluées, éventuellement, le test de rinçage comprenant au moins l'un des éléments suivants :
- un rinçage rapide automatisé, dans lequel l'ouverture du réservoir sous pression et/ou l'arrêt brusque du flux sont opérés automatiquement à l'aide d'un moyen de rinçage automatisé capable d'induire un rinçage qui peut être une injection courte dans le temps d'un liquide, de préférence une solution saline, avec pression dans la ligne artérielle,
- un rinçage rapide manuel, dans lequel une instruction d'effectuer l'ouverture manuelle du réservoir sous pression et/ou l'arrêt brusque du flux est signalée à un opérateur, et
- un test manuel de tapotement, le test de tapotement comprenant une augmentation une ou plusieurs fois de la pression sanguine en appliquant une pression augmentée au travers de la ligne artérielle, par exemple en tapotant sur un tube flexible raccordé à la ligne artérielle avec préférentiellement un relâchement soudain de la pression appliquée, le test manuel de tapotement comprenant la signalisation à l'opérateur d'une instruction de réalisation du test manuel de tapotement.

15. Système de notification d'un événement de coagulation dans une ligne artérielle (1) d'un être humain (2) selon la revendication 14, le système étant en outre configuré pour, après détermination d'un événement de coagulation, vérifier que l'événement de coagulation déterminé est effectivement un événement de coagulation vrai basé sur l'amortissement évalué et la fréquence naturelle du système artériel, en particulier, le système étant en outre configuré pour, dans le cas d'un événement de coagulation vrai vérifié, exécuter un rinçage automatisé, la ligne artérielle comprenant un moyen de rinçage automatisé capable d'induire un rinçage qui peut être une injection courte dans le temps d'un liquide, de préférence une solution saline, avec pression dans la ligne artérielle afin de libérer la ligne artérielle du sang coagulé.
